# EUROPÄISCHE PATENTSCHRIFT

(11) **EP 2 675 404 B1**
(45) Veröffentlichungstag und Bekanntmachung des Hinweises auf die Patenterteilung: **30.09.2015**
(21) Anmeldenummer: 12703513.7
(22) Anmeldetag: 07.02.2012
(51) Int. Cl.: A61F 2/90, D04C 1/08

(54) **MEDIZINISCHE VORRICHTUNG MIT EINEM EXPANDIERBAREN GITTERGEFLECHT**
MEDICAL DEVICE HAVING AN EXPANDABLE BRAIDED MESH
DISPOSITIF MÉDICAL DOTÉ D'UNE MAILLE TRESSÉE EXTENSIBLE

(30) Priorität: 14.02.2011 DE 102011011150
(43) Veröffentlichungstag der Anmeldung: 25.12.2013
(73) Patentinhaber: Acandis GmbH & Co. KG, 76327 Pfinztal (DE)
(72) Erfinder: CATTANEO, Giorgio, 76199 Karlsruhe (DE); BAIDINGER, Otto, 75175 Pforzheim (DE)
(74) Vertreter: Kilchert, Jochen
(86) Internationale Anmeldenummer: PCT/EP2012/052008
(87) Internationale Veröffentlichungsnummer: WO 2012/110355

(56) Entgegenhaltungen:
- WO-A1-2008/130530
- US-A1- 2009 054 972
- US-A1- 2009 177 268

## Beschreibung

Die Erfindung betrifft eine medizinische Vorrichtung gemäß dem Oberbegriff des Patentanspruchs 1. Eine derartige medizinische Vorrichtung ist beispielsweise aus WO 2008/130530 A1 bekannt.

WO 2008/130530 A1 offenbart eine medizinische Vorrichtung, insbesondere einen Stent, der ein Gittergeflecht aus miteinander verflochtenen bzw. sich kreuzenden Drähten umfasst. Die einzelnen Drähte sind an einem axialen Ende des Stents umgelenkt und in das Gittergeflecht zurückgeführt. Auf diese Weise sind an dem axialen Stentende Schlaufen gebildet. Aufgrund der Schlaufen weist das axiale Stentende keine offenen Drahtenden auf und ist daher im Wesentlichen atraumatisch ausgebildet.

Der Stent gemäß WO 2008/130530 A1 ist expandierbar. Im expandierten Zustand sind die in Umfangsrichtung benachbart angeordneten Schlaufen des Stentendes zueinander axial versetzt angeordnet. Jede zweite Schlaufe ragt also weiter über das Gittergeflecht des Stents hinaus als die jeweils dazwischen angeordnete Schlaufe.

Dabei ist die Radialkraft nicht zwingend über die gesamte Längserstreckung der Gitterstruktur gleich. Bei Gittergeflechten, die an den axialen Enden offene Drahtenden aufweisen, hat sich beispielsweise gezeigt, dass die Radialkraft an den axialen Enden geringer als im Inneren der Gitterstruktur ist. Im expandierten Zustand nimmt das Geflecht daher eine im Wesentlichen zigarrenartige Form ein. Die Schlaufen an den axialen Enden, wie bei dem Stent gemäß WO 2008/130530 A1 vorgesehen, erhöhen demgegenüber die Radialkraft an den axialen Enden. Bei besonders feinmaschigen Geflechten oder bei Geflechten mit vergleichsweise dicken Drähten entsteht durch die als Schlaufen umgelenkten Drähte im komprimierten Zustand eine erhöhte Kraft, die auf eine Katheterinnenwand wirkt. Dadurch wird die Reibungskraft zwischen dem Stent und der Katheterinnenwand eines Zufuhrsystems erhöht, so dass die Zufuhr derartiger Stents mit schlaufenartig gestalteten axialen Enden erschwert ist.

Der Erfindung liegt die Aufgabe zugrunde, eine medizinische Vorrichtung abzugeben, die an den Längsenden eine ausreichende Radialkraft aufweist und gleichzeitig eine einfache Zufuhr über ein Zuführsystem ermöglicht.

Erfindungsgemäß wird diese Aufgabe durch den Gegenstand des Patentanspruchs 1 gelöst.

Die Erfindung beruht auf dem Gedanken, eine medizinische Vorrichtung mit einem expandierbaren Gittergeflecht aus sich überkreuzenden Drähten anzugeben, die an wenigstens einem Längsende des Gittergeflechts Schlaufen bilden. Dabei bilden ein erster Draht eine erste Schlaufe und ein zweiter Draht eine zweite Schlaufe. Die erste Schlaufe und die zweite Schlaufe sind in einem expandierten Zustand des Gittergeflechts zumindest abschnittsweise deckungsgleich aufeinander angeordnet. In einem komprimierten Zustand des Gittergeflechts sind die erste Schlaufe und die zweite Schlaufe voneinander beabstandet angeordnet.

Die Erfindung hat den Vorteil, dass durch die im komprimierten Zustand des Gittergeflechts beabstandet angeordneten ersten und zweiten Schlaufen der Querschnittsdurchmesser des Längsendes des Gittergeflechts im komprimierten Zustand reduziert ist. Die Radialkraft des Längsendes des Gittergeflechts im komprimierten Zustand ist reduziert, so dass die für die Zufuhr der medizinischen Vorrichtung über ein Zuführsystem erforderliche Kraft verringert wird. Die erfindungsgemäße medizinische Vorrichtung lässt sich daher leicht und mit geringem Kraftaufwand an den Bestimmungsort führen. Im expandierten Zustand liegen die ersten und zweiten Schlaufen hingegen zumindest abschnittsweise deckungsgleich aufeinander, so dass sich die von den einzelnen Schlaufen bewirkte Radialkraft summiert. Im expandierten Zustand weist das Gittergeflecht an seinem Längsende also eine erhöhte Radialkraft auf. Vorzugsweise sind die ersten und zweiten Schlaufen derart dimensioniert, dass sich die ersten und zweiten Schlaufen in einem Expansionszustand deckungsgleich überlappen, der sich am Bestimmungsort, beispielsweise einem Blutgefäß, einstellt. Dieser expandierte Zustand, der auch als Implantationszustand bezeichnet wird, ist üblicherweise kleiner als der vollständig expandierte Zustand, den das Gittergeflecht bei der Herstellung einnimmt. Im vollständig expandierten Zustand wirken auf das Gittergeflecht bzw. allgemein die medizinische Vorrichtung im Wesentlichen keine äußeren Kräfte. Das Gittergeflecht ist also im vollständig expandierten Zustand zwängungsfrei expandiert.

Im Allgemeinen können sich die erste Schlaufe und die zweite Schlaufe in beliebigen expandierten Zuständen überlappen bzw. abschnittsweise aufeinander angeordnet sein. Der in diesem Zusammenhang genannte expandierte Zustand bezieht sich also nicht nur auf den vollständig expandierten Zustand, sondern auch auf teilexpandierte Zustände, beispielsweise den Implantationszustand. Die deckungsgleiche Anordnung der ersten und zweiten Schlaufe aufeinander bezieht sich sowohl auf eine punktuelle Überdeckung, als auch insbesondere auf eine linienförmige Überdeckung. Die erste und die zweite Schlaufe können sich also prinzipiell im expandierten Zustand kreuzen oder derart angeordnet sein, dass sich die Schlaufen im expandierten Zustand entlang eines Schlaufenabschnitts überdecken, so dass sich insgesamt eine linienförmige Überlappung einstellt.

Bei einer bevorzugten Ausführungsform der erfindungsgemäßen medizinischen Vorrichtung sind die erste Schlaufe und die zweite Schlaufe des Gittergeflechts in Längsrichtung des Gittergeflechts im komprimierten Zustand hintereinander angeordnet. Mit anderen Worten weist das Gittergeflecht vorzugsweise eine Doppelschlaufe oder Mehrfachschlaufe an seinem Längsende auf. Insbesondere weist das Gittergeflecht an einem oder beiden Längsenden mehrere erste Schlaufen auf, denen jeweils eine zweite Schlaufe bzw. Innenschlaufe zugeordnet ist. Vorzugsweise sind alle Endschlaufen eines Längsendes als Doppel- oder Mehrfachschlaufe ausgebildet. In Längsrichtung des Gittergeflechts sind also wenigstens zwei Schlaufen direkt hintereinander bzw. in Längsrichtung zueinander versetzt angeordnet. Die Mehrfachschlaufe kann auch mehr als zwei hintereinander angeordnete Schlaufen umfassen. Dies gilt für den komprimierten Zustand. Durch die Expansion, also die Aufweitung des Gittergeflechts in Querrichtung, insbesondere in Umfangsrichtung bei rotationssymmetrischen Gittergeflechten, verschieben sich die erste und die zweite Schlaufe relativ zueinander, so dass sich die erste und die zweite Schlaufe in einem expandierten Zustand, vorzugsweise im Implantationszustand, zumindest abschnittsweise deckungsgleich überlappen bzw. aufeinander anordnen. Durch die Anordnung der Schlaufen in Längsrichtung des Gittergeflechts hintereinander wird erreicht, dass die Anzahl der Schlaufen in Querrichtung des Gittergeflechts reduziert ist. Das Längsende des Gittergeflechts weist also insgesamt eine relativ kleine Anzahl von Schlaufen in Querrichtung auf, so dass sich das Gittergeflecht sehr klein komprimieren lässt. Dennoch wird durch die im komprimierten Zustand in Längsrichtung des Gittergeflechts dahinter liegenden Schlaufen im expandierten Zustand eine Radialkrafterhöhung bewirkt. Insgesamt trägt die in Längsrichtung des Gittergeflechts versetzte Anordnung der ersten Schlaufe und der zweiten Schlaufe im komprimierten Zustand zu einer verbesserten Komprimierbarkeit und einer Reduktion der Radialkraft im komprimierten Zustand bei, so dass die Zuführung der medizinischen Vorrichtung durch ein Zuführsystem, insbesondere einen Katheter, erleichtert ist.

Die Schlaufen können einstückig durch jeweils einen einzigen Draht, der am Längsende des Gittergeflechts umgelenkt ist, oder durch jeweils zwei Drähte gebildet sein, die am Längsende des Gittergeflechts miteinander verbunden sind. Die einstückige Ausbildung der Schlaufen durch jeweils einen einzigen Draht hat den Vorteil, dass die Schlaufen eine erhöhte Flexibilität aufweisen. Bei der einstückigen Ausbildung können die Schlaufen aus einem einzigen Draht gebildet sein, der am Längsende des Gittergeflechts umgelenkt und in das Gittergeflecht zurückgeführt ist. Bei der zweistückigen Ausbildung der Schlaufen sind zwei Drähte im Bereich des Längsendes des Gittergeflechts zusammengeführt und miteinander verbunden. Diese Variante zeichnet sich durch eine einfache Herstellbarkeit aus. Die Verbindung der Drähte kann beispielsweise durch Löten, Schweißen oder durch Hülsen erfolgen, die mit den Drähten verbunden, insbesondere auf die Drähte gecrimpt, werden. Die Hülsen können beispielsweise als Röntgenmarker fungieren. Dadurch wird die Positionierbarkeit der medizinischen Vorrichtung verbessert. Es ist auch möglich, dass die medizinische Vorrichtung ein Gittergeflecht aufweist, an dessen Längsende einerseits einstückig ausgebildete Schlaufen und andererseits Schlaufen ausgebildet sind, die durch die Verbindung zweier Drähte hergestellt sind. Mit anderen Worten ist eine Kombination von Schlaufen, die einstückig durch jeweils einen einzigen Draht gebildet sind mit Schlaufen, die durch die Verbindung zweier Drähte gebildet sind, möglich.

Vorzugsweise ist das Gittergeflecht der medizinischen Vorrichtung rotationssymmetrisch, insbesondere zylinderförmig, ausgebildet. Dabei können die Drähte spiralförmig bzw. schraubenförmig um eine gemeinsame Rotationsachse gewunden sein. Die rotationssymmetrische, insbesondere zylinderförmige, Gestaltung des Gittergeflechts ermöglicht vorteilhaft die Verwendung des Gittergeflechts für Stents, Stent-Grafts, Flowdiverter, Thrombectomie-Devices, Filter oder andere endoluminale Implantate oder Instrumente.

Bei einer bevorzugten Ausführungsform der erfindungsgemäßen medizinischen Vorrichtung verlaufen der erste Draht und der zweite Draht innerhalb des Gittergeflechts parallel zueinander. Der erste Draht und der zweite Draht können insbesondere dieselbe Windungsrichtung um eine Längsachse des Gittergeflechts aufweisen. Der erste Draht und der zweite Draht sind dabei innerhalb des Gittergeflechts beabstandet zueinander angeordnet. Insbesondere bilden der erste Draht und der zweite Draht kein Drahtbündel, das gemeinsam mit weiteren Drähten oder Drahtbündeln verflochten ist. Vielmehr sind der erste Draht und der zweite Draht selbstständig innerhalb des Gittergeflechts angeordnet. Die Parallelität des ersten Drahts und des zweiten Drahts bezieht sich dabei auf die Anordnung entlang des Umfangs des Gittergeflechts. In radialer Richtung bezogen auf die Längsachse des Gittergeflechts können der erste Draht und der zweite Draht stellenweise versetzt sein. Insbesondere ist vorgesehen, dass der erste Draht einen weiteren quer verlaufenden Draht überkreuzt, wogegen der zweite Draht denselben quer verlaufenden Draht unterkreuzt. Der erste Draht und der zweite Draht sind also mit weiteren Drähten des Gittergeflechts unterschiedlich verflochten.

Der erste Draht bildet die erste Schlaufe und der zweite Draht die zweite Schlaufe, so dass sich der erste Draht und der zweite Draht im Bereich der Schlaufe zumindest abschnittsweise überlagern, wenn das Gittergeflecht einen expandierten Zustand, insbesondere den Implantationszustand oder den vollständig expandierten Zustand (Ruhezustand), einnimmt. Im expandierten Zustand können sich der erste Draht und der zweite Draht im Bereich der ersten und zweiten Schlaufe, insbesondere im Bereich der Überlagerung, berühren und einen Kontaktbereich bilden. Im komprimierten Zustand sind der erste Draht und der zweite Draht zumindest im Bereich der Schlaufen, insbesondere der ersten Schlaufe und der zweiten Schlaufe, in axialer Richtung voneinander beabstandet angeordnet. Aufgrund der radialen Komprimierung kann dennoch ein Kontakt der Schlaufen in Umfangsrichtung bestehen.

Die Drähte im Bereich der Schlaufen können jeweils mit weiteren Drähten Kreuzungspunkte bilden, die Schlaufenendpunkte festlegen. Innerhalb des Gittergeflechts überkreuzen die Drähte, insbesondere der erste Draht und der zweite Draht, jeweils weitere Drähte des Gittergeflechts. Die Drähte bilden also mit den weiteren Drähten Kreuzungspunkte. Am Längsende des Gittergeflechts bilden der erste Draht und der zweite Draht ausgehend vom letzten Kreuzungspunkt des ersten bzw. zweiten Drahts mit einem weiteren Draht die erste oder zweite Schlaufe. Der Abschnitt der Drähte, der am Längsende des Gittergeflechts über den letzten Kreuzungspunkt dieses Drahtes mit einem weiteren Draht vorsteht, bildet also die Schlaufe im Sinne der Erfindung. Die letzten Kreuzungspunkte des Gittergeflechts bzw. des ersten oder zweiten Drahts mit einem weiteren Draht, bestimmen die Schlaufenendpunkte. Jede Schlaufe weist zwei Schlaufenendpunkte auf.

Vorzugsweise ist der Abstand der Schlaufenendpunkte der ersten Schlaufe kleiner als der Abstand der Schlaufenendpunkte der zweiten Schlaufe. Der Abstand der Schlaufenendpunkte wird in Querrichtung des Gittergeflechts, insbesondere in Umfangsrichtung des rotationssymmetrischen Gittergeflechts, ermittelt. Mit anderen Worten ist die erste Schlaufe vorzugsweise schmaler als die zweite Schlaufe ausgebildet. Durch die unterschiedlichen Geometrien der ersten und zweiten Schlaufe wird der vorteilhafte Effekt erreicht, dass sich die Schlaufen bei der Expansion des Gittergeflechts bzw. bei der Komprimierung des Gittergeflechts relativ zueinander bewegen. Auf diese Weise kann sich im komprimierten Zustand die versetzte Anordnung und in einem expandierten Zustand, insbesondere dem Implantationszustand, die aufeinander liegende Anordnung der Schlaufen einstellen.

Die Erfindung wird im Folgenden anhand von Ausführungsbeispielen unter Bezugnahme auf die beigefügten, schematischen Zeichnungen näher erläutert. Darin zeigen:
- Figur 1:: eine Abwicklung eines rohrförmigen Gittergeflechts der erfindungsgemäßen medizinischen Vorrichtung nach einem bevorzugten Ausführungsbeispiel im expandierten Zustand;
- Figur 2:: die Vorrichtung gemäß Figur 1 im komprimierten Zustand;
- Figur 3a:: eine Draufsicht auf eine einzelne Masche eines Gittergeflechts im expandierten Zustand;
- Figur 3b:: die Masche gemäß Figur 3a im komprimierten Zustand;
- Figur 4a:: eine Draufsicht auf zwei ineinander liegende Maschen eines Gittergeflechts im expandierten Zustand;
- Figur 4b:: die Maschen gemäß Figur 4a im komprimierten Zustand; und
- Figur 5:: das Gittergeflecht gemäß Figur 1, wobei zwei sich überlagernde Schlaufen hervorgehoben sind.

Die erfindungsgemäße medizinische Vorrichtung kann als Stent, Stent-Graft, Flowdiverter, Thrombectomie-Device oder Thrombenfilter ausgebildet sein. Bevorzugt ist die Ausbildung als Stent, wobei die medizinische Vorrichtung grundsätzlich medizinische Implantate oder medizinische Instrumente bilden kann, die über ein Zuführsystem, insbesondere einen Katheter, in den menschlichen Körper, vorzugsweise minimalinvasiv, einführbar sind.

Die medizinische Vorrichtung umfasst ein expandierbares Gittergeflecht 10, das aus Drähten 11, 12 gebildet ist. Zur Bildung des Gittergeflechts 10 sind die Drähte 11, 12 verflochten, wobei sich die Drähte 11, 12 kreuzen und Kreuzungspunkte 16 bilden. Das Gittergeflecht ist vorzugsweise rotationssymmetrisch, insbesondere zylinderförmig, ausgebildet. Das Gittergeflecht 10 weist also eine Rotationsachse bzw. Längsachse auf, um die die Drähte 11, 12 spiralförmig gewunden sind. An wenigstens einem axialen Ende bzw. Längsende 15 des Gittergeflechts 10 bilden die Drähte 11, 12 Schlaufen 13, 14. Dabei sind die Drähte 11, 12 am Längsende 15 des Gittergeflechts 10 umgelenkt und in die Geflechtstruktur des Gittergeflechts 10 zurückgeführt. Alternativ können zwei Drähte 11, 12 am Längsende 15 miteinander verbunden werden, so dass sich eine Schlaufe 13, 14 bildet.

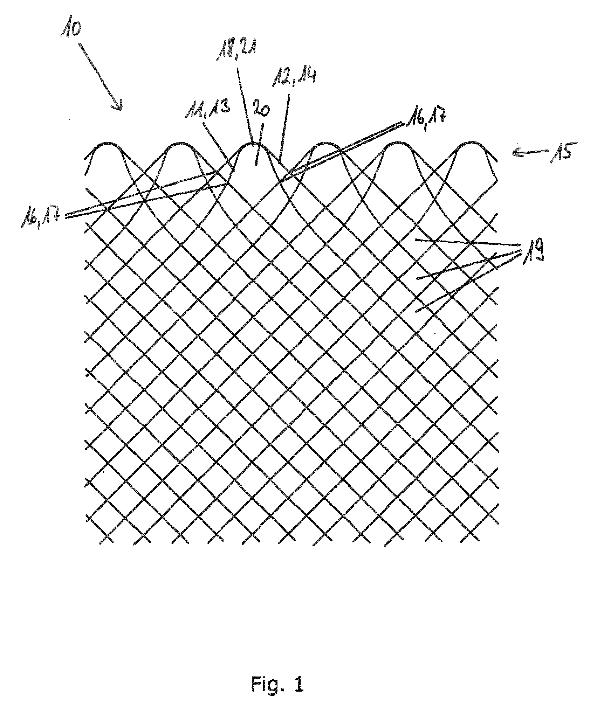

Die Drähte 11, 12 begrenzen Maschen 19 des Gittergeflechts 10. Eine Masche 19 ist insgesamt durch 4 Drähte 11, 12 begrenzt, die gemeinsam vier Kreuzungspunkte 16 bilden. Die Maschen 19 weisen vorzugsweise eine rautenförmige Geometrie auf. Im Unterschied zu den Maschen 19 werden die Schlaufen 13, 14 durch einen einzigen Draht 11, 12 oder höchstens zwei Drähte 11, 12, die miteinander verbunden sind, gebildet. Wie in Figur 1 erkennbar ist, weisen die Schlaufen 13, 14 zwei Seiten auf, die entweder durch einzigen Draht 11, 12 oder zwei miteinander verbundene Drähte 11, 12 gebildet sind. Im Übrigen sind die Schlaufen in Richtung des Gittergeflechts 10 offen. Im Unterschied dazu sind die Maschen 19 des Gittergeflechts 10 geschlossen, also allseitig durch Drähte 11, 12 begrenzt. Am Längsende 15 des Gittergeflechts 10 bilden die Drähte 11, 12 jeweils Schlaufen 13, 14. Gleichzeitig bilden die Drähte 11, 12 am Längsende 15 des Gittergeflechts 10 mit weiteren Drähten Endmaschen 20, die sich von den Maschen 19 des Gittergeflechts 10 dadurch unterscheiden, dass sie einerseits durch zwei sich kreuzende Drähte 11, 12 und andererseits durch eine Schlaufe 13, 14 begrenzt sind. Im Unterschied zu den Maschen 19 des Gittergeflechts, die jeweils vier Kreuzungspunkte 16 umfassen, weisen die Endmaschen 20 des Gittergeflechts 10 drei Kreuzungspunkte 16 auf. Dennoch sind die Endmaschen 20 am Längsende 15 des Gittergeflechts 10 weitgehend rautenförmig ausgebildet, da die Drähte 11, 12 der Schlaufen 13, 14 am Längsende 15 umgelenkt und zurückgeführt sind. Die Drähte 11, 12 sind am Längsende 15 des Gittergeflechts 10 derart umgelenkt, dass sich die Windungsrichtung der spiralförmig gewundenen Drähte bezüglich der um die Längsachse des Gittergeflechts 10 ändert.

Figur 1 zeigt das Gittergeflecht 10 im expandierten Zustand. Darin ist zu erkennen, dass am Längsende 15 mehrere Schlaufen 13, 14 vorgesehen sind, die in Querrichtung bzw. Umfangsrichtung UR des Gittergeflechts 10 benachbart angeordnet und voneinander beabstandet sind. Das Längsende 15 gemäß Figur 1 weist Mehrfachschlaufen, insbesondere Doppelschlaufen auf, wobei eine erste Schlaufe 13 durch einen ersten Draht 11 und eine zweite Schlaufe 14 durch einen zweiten Draht 12 gebildet ist. Die erste Schlaufe 13 und die zweite Schlaufe 14 überlagern sich im expandierten Zustand. Vorzugsweise überlagern sich die erste Schlaufe 13 und die zweite Schlaufe 14 in einem Implantationszustand, in dem das Gittergeflecht 10 einen geringfügig kleineren Querschnittsdurchmesser aufweist, als im vollständig expandierten Zustand bzw. Herstellzustand. Dies kann durch entsprechende Dimensionierung der ersten Schlaufe 13 und der zweiten Schlaufe 14 erfolgen. Konkret wird im expandierten Zustand gemäß Figur 1 ein Kontaktbereich 18 gebildet, in dem die erste Schlaufe 13 auf der zweiten Schlaufe 14 aufliegt. Zumindest im Kontaktbereich 18 sind die erste Schlaufe 13 und die zweite Schlaufe 14 deckungsgleich aufeinander liegend angeordnet. Dabei ist nicht ausgeschlossen, dass zwischen den Schlaufen 13, 14 ein Spalt in radialer Richtung bezogen auf die Längsachse des Gittergeflechts 10 besteht. Wesentlich ist, dass die Schlaufen 13, 14 einer Doppelschlaufe oder Mehrfachschlaufe in einem expandierten Zustand auf derselben axialen Höhe angeordnet sind.

Die erste Schlaufe 13 ist zumindest im expandierten Zustand des Gittergeflechts schmaler ausgebildet als die zweite Schlaufe 14. Insbesondere ist der Abstand zwischen zwei Schlaufenendpunkten 17 der ersten Schlaufe 13 kleiner als der Abstand zweier Schlaufenendpunkte 17 der zweiten Schlaufe 14. Die Schlaufenendpunkte 17 entsprechen den Kreuzungspunkten 16 des ersten Drahts bzw. zweiten Drahts 12 mit weiteren Drähten des Gittergeflechts 10, wobei die die Schlaufenendpunkte 17 bildenden Kreuzungspunkte 16 dem Längsende 15 am nächsten liegen. Mit anderen Worten weist das Gittergeflecht 10 Kreuzungspunkte 16 auf, die ausgehend vom Längsende 15 die abschließenden bzw. letzten Kreuzungspunkte 16 des Gittergeflechts 10 bilden. Die letzten Kreuzungspunkte 16 entsprechen den Schlaufenendpunkten 17, wie in Figur 1 erkennbar ist. Der Abstand der Schlaufenendpunkte 17 wird in Umfangsrichtung UR des Gittergeflechts 10 ermittelt.

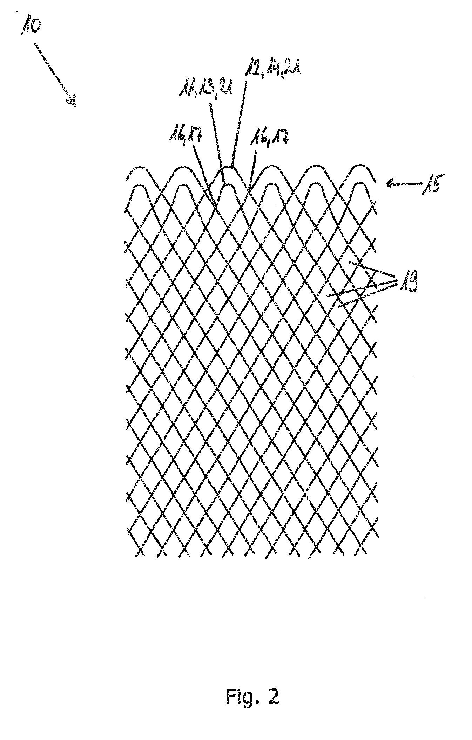

Der unterschiedliche Abstand zwischen den Schlaufenendpunkten 17 der ersten Schlaufe 13 und der zweiten Schlaufe 14 bzw. allgemein die unterschiedliche Geometrie der ersten Schlaufe 13 und der zweiten Schlaufe 14 bewirken eine Relativbewegung zwischen den Schlaufen 13, 14 bei der Überführung des Gittergeflechts 10 vom expandierten Zustand in den komprimierten Zustand. Insbesondere wird bei der Komprimierung des Gittergeflechts 10 der Kontaktbereich 18 der Schlaufen 13, 14 aufgelöst, so dass sich die erste Schlaufe 13 von der zweiten Schlaufe 14 entfernt. Im komprimierten Zustand des Gittergeflechts 10 sind die erste Schlaufe 13 und die zweite Schlaufe 14 beabstandet zueinander angeordnet, wie in Figur 2 gezeigt ist. Bei der Überführung von dem expandierten Zustand in den komprimierten Zustand verringert sich der Abstand der Schlaufenendpunkte 17 einer Schlaufe 13, 14. Gleichzeitig bewegt sich eine Schlaufenspitze 21 der ersten Schlaufe 13 bzw. der zweiten Schlaufe 14 in Längsrichtung LR des Gittergeflechts 10. Aufgrund der unterschiedlichen Schlaufengeometrien weisen die Schlaufenspitzen 21 der ersten Schlaufe 13 und der zweiten Schlaufe 14 unterschiedliche Bewegungsgeschwindigkeiten in Längsrichtung des Gittergeflechts 10 auf, so dass sich die erste Schlaufe 13 und die zweite Schlaufe 14 voneinander entfernen. Im komprimierten Zustand gemäß Figur 2 besteht kein Kontakt zwischen dem ersten Draht 11 und dem zweiten Draht 12. Hingegen berühren sich der erste Draht 11 und der zweite Draht 12 im expandierten Zustand gemäß Figur 1 am Längsende 15, insbesondere im Kontaktbereich 18.

In Figur 2 ist auch gut erkennbar, dass die Relativbewegung zwischen den Schlaufen 12, 13, die im expandierten Zustand eine Mehrfachschlaufe bilden, in Längsrichtung LR des Gittergeflechts 10, also parallel zur Längsachse des Gittergeflechts 10, erfolgt.

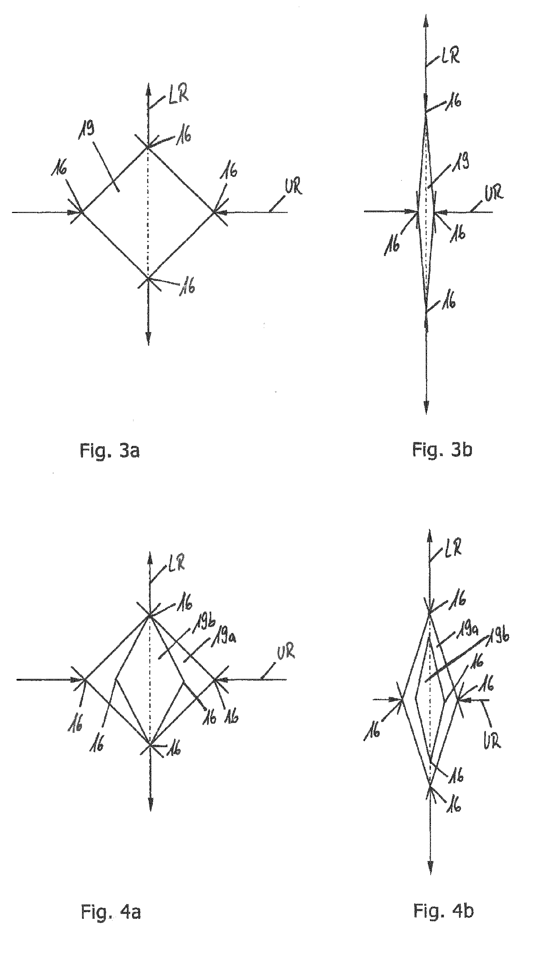

Der Mechanismus, der zu der Relativbewegung zwischen den ersten und zweiten Schlaufen 13, 14 führt, wird nachfolgend anhand der Figuren 3a-4b näher erläutert:
Das Gittergeflecht 10, insbesondere ein rotationssymmetrisches bzw. zylinderförmiges oder rohrförmiges Gittergeflecht 10, umfasst üblicherweise mehrere Maschen 19. Im expandierten Zustand, wie in Figur 3a dargestellt, kann eine einzelne Masche 19, die im Wesentlichen rautenförmig ausgebildet ist, zwei im Wesentlichen gleich große Diagonalen aufweisen. Bei einer Komprimierung des Gittergeflechts 10 werden die in Umfangsrichtung UR benachbart angeordneten Kreuzungspunkte 16 aufeinander zugeführt, wodurch gleichzeitig bewirkt wird, dass die in Längsrichtung LR benachbart angeordneten Kreuzungspunkte 16 der Masche 19 voneinander entfernt werden (Figur 3b). Die Verkleinerung der Diagonalen in Querrichtung bzw. Umfangsrichtung UR des Gittergeflechts 10 bewirkt also eine Vergrößerung der Diagonalen in Längsrichtung LR des Gittergeflechts 10. Die Bewegung der Kreuzungspunkte 16 in Umfangrichtung und Längsrichtung ist in den Figuren 3a und 3b durch entsprechende Pfeile verdeutlicht. Die Komprimierung des Gittergeflechts 10 bewirkt, dass die einzelnen Maschen 19 in Umfangsrichtung UR zusammengedrückt und in Längsrichtung LR gestreckt werden.

Werden zwei Maschen 19, die im expandierten Zustand unterschiedliche Dimensionsverhältnisse aufweisen, überlagert bzw. übereinander gelegt, ergibt sich zwischen dem Kreuzungspunkt 16 der beiden Maschen 19 bei der Komprimierung des Gittergeflechts 10 eine Relativbewegung. Figur 4a zeigt zwei Maschen 19, wobei eine erste Masche 19a analog zu der Masche 19 gemäß Figur 3a im Wesentlichen gleich lange Diagonalen aufweist. Eine zweite Masche 19b weist eine Diagonale in Umfangsrichtung des Gittergeflechts 10 auf, die kleiner ist als eine Diagonale in Längsrichtung LR des Gittergeflechts 10. Die Diagonale in Längsrichtung LR der zweiten Masche 19b ist gleich lang wie die Diagonale in Längsrichtung LR der ersten Masche 19a. Die in Längsrichtung LR benachbart angeordneten Kreuzungspunkte 16 der ersten Masche 19a und der zweiten Masche 19b überlagern sich bzw. sind deckungsgleich aufeinander angeordnet. Eine Komprimierung des Gittergeflechts 10 bewirkt nun eine Verkürzung der in Umfangsrichtung UR ausgerichteten Diagonalen der ersten Masche 19a und der zweiten Masche 19b. Dabei wird gegenüber der Diagonale der zweiten Masche 19b längere Diagonale der ersten Masche 19a, die sich in Umfangsrichtung UR des Gittergeflechts 10 erstreckt, stärker verkürzt als die Diagonale der zweiten Masche 19b, die sich in Querrichtung des Gittergeflechts 10 erstreckt. Das bewirkt wiederum, dass sich die erste Masche 19a stärker streckt als die zweite Masche 19b (Figur 4b). Mit anderen Worten wird der Abstand der in Längsrichtung LR des Gittergeflechts 10 benachbart angeordneten Kreuzungspunkte 16 der ersten Masche 19a stärker vergrößert als der Abstand der Kreuzungspunkte 16 der zweiten Masche 19b, die in Längsrichtung des Gittergeflechts 10 benachbart angeordnet sind. Die Kreuzungspunkte 16 der beiden ineinander angeordneten Maschen 19, die in Längsrichtung des Gittergeflechts 10 benachbart angeordnet sind, entfernen sich somit voneinander, wenn das Gittergeflecht 10 in den komprimierten Zustand überführt wird.

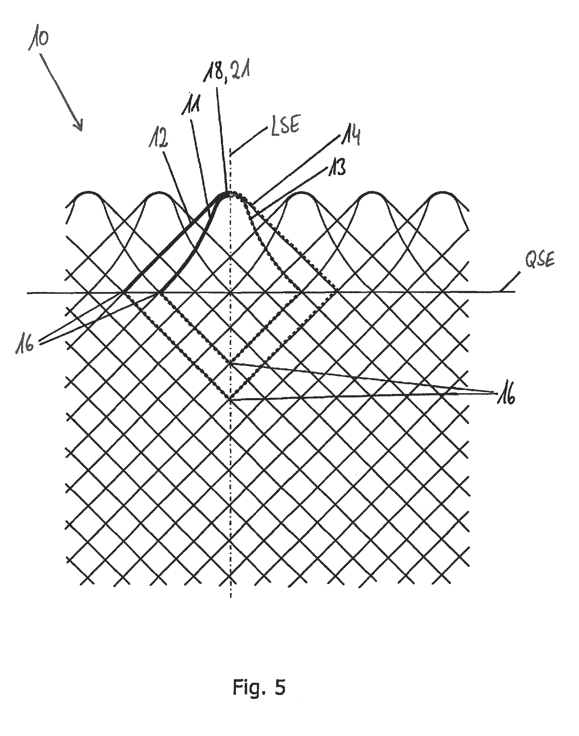

Das im Zusammenhang mit den Figuren 3a-4b erläuterte Prinzip ist auf die Schlaufen 13, 14 übertragbar. In Figur 5 ist eine Mehrfachschlaufe hervorgehoben, die aus zwei Schlaufen, einer ersten Schlaufe 13 und einer zweiten Schlaufe 14, gebildet ist. Die erste Schlaufe 13 ist durch einen ersten Draht 11 und die zweite Schlaufe 14 durch einen zweiten Draht 12 gebildet. In Umfangsrichtung UR des Gittergeflechts 10 sind am Längsende 15 mehrere Mehrfachschlaufen benachbart zueinander angeordnet. Figur 5 zeigt den expandierten Zustand des Gittergeflechts 10, wobei sich die Schlaufenspitzen 21 überlappen bzw. aufeinander angeordnet sind derart, dass sich ein Kontaktbereich 18 bildet. Im Kontaktbereich 18 berühren sich der erste Draht 11 und der zweite Draht 12. Der erste Draht 11 und der zweite Draht 12 sind im Kontaktbereich 18 - in radialer Richtung des Gittergeflechts 10 - betrachtet aufeinander angeordnet bzw. aufeinander aufliegend angeordnet.

Der erste Draht 11 und der zweite Draht 12 sind in Figur 5 abschnittsweise durch eine verdickte Liniendarstellung kenntlich gemacht. Darin ist auch zu erkennen, dass der erste Draht 11 und der zweite Draht 12 innerhalb des Gittergeflechts 10 im Wesentlichen parallel zueinander verlaufen. Insbesondere ist vorgesehen, dass sich der erste Draht 11 und der zweite Draht 12 innerhalb des Gittergeflechts 10 nicht überkreuzen. Der erste Draht 11 bildet also mit dem zweiten Draht 12 keinen Kreuzungspunkt 16. Vielmehr überlagern sich der erste Draht 11 und der zweite Draht 12 im expandierten Zustand des Gittergeflechts 10 im Kontaktbereich 18. Die in Figur 5 dargestellten, gepunkteten Linien veranschaulichen die Ähnlichkeit der Schlaufenkonfiguration mit den Maschen 19 gemäß Figur 3a-4b. Der erste Draht 11, der die erste Schlaufe 13 bildet und der zweite Draht 12, der die zweite Schlaufe 14 bildet, überkreuzen sich jeweils innerhalb des Gittergeflechts 10 mit weiteren Drähten und bilden Kreuzungspunkte 16. Dadurch werden Zellen bzw. Maschen 19 gebildet, die durch die punktierte Linie in Figur 5 hervorgehoben sind. Das Verhalten der durch die punktierten Linien dargestellten Zellen des Gittergeflechts 10 bei der Überführung des Gittergeflechts 10 vom expandierten Zustand in den komprimierten Zustand entspricht dem Verhalten der Maschen 19 gemäß Figuren 3a-4b.

In Figur 5 ist überdies eine axiale Querschnittsebene QSE eingezeichnet, die sich durch in Umfangsrichtung UR unmittelbar benachbart angeordnete Kreuzungspunkte 16 erstreckt. Die Kreuzungspunkte 16, die in der Querschnittsebene QSE angeordnet sind, bewegen sich bei einer Komprimierung des Gittergeflechts 10 aufeinander zu. Mit anderen Worten verringert sich der Abstand der Kreuzungspunkte 16 einer Querschnittsebene QSE bei der Komprimierung des Gittergeflechts 10. Dadurch wird erreicht, dass sich die in einer Längsschnittebene LSE des Gittergeflechts unmittelbar benachbart angeordneten Kreuzungspunkte 16 voneinander entfernen. Die Schlaufen 13, 14, die in der Längsschnittebene LSE angeordnet sind, entfernen sich somit ebenfalls voneinander. Die Längsschnittebene LSE ist in Figur 5 durch die strichpunktierte Linie dargestellt.

Die axiale Querschnittsebene QSE begrenzt auch die parallele Anordnung des ersten und zweiten Drahts 11, 12 im Gittergeflecht 10. Wie in Fig. 5 gut zu erkennen ist, konvergieren die Drähte 11, 12 am Längsende 15 des Gittergeflechts 10 zueinander. Insbesondere weist der erste Draht 11, der die innere Schlaufe 13 bildet, einen gekrümmten Verlauf auf und nähert sich dem zweiten Draht 12, der die äußere Schlaufe 14 bildet, an. Der gekrümmte Verlauf des ersten Drahts 11 beginnt auf Höhe der axialen Querschnittsebene QSE und erstreckt sich bis zur Schlaufenspitze 21. Mit anderen Worten ändert sich der Flechtwinkel des ersten Drahts 11 ausgehend von der Querschnittsebene QSE bis hin zur Schlaufenspitze 21 kontinuierlich.

Als Flechtwinkel wird im Rahmen der Anmeldung der spitze Winkel bezeichnet, der sich zwischen der Längsschnittebene LSE und einem Draht 11, 12 einstellt. Dabei umfasst die Längsschnittebene LSE auch die Längsachse des Gittergeflechts 10. Ausgehend von der Querschnittsebene QSE in Richtung des Geflechtinneren verlaufen der erste Draht 11 und der zweite Draht 12 parallel zueinander, weisen also denselben Flechtwinkel auf.

Für den Verlauf des ersten Drahts 11 zwischen der Querschnittsebene QSE und der Schlaufenspitze 21 lässt sich ein Durchschnittsflechtwinkel beschreiben, wobei der Durchschnittsflechtwinkel zwischen einer gedachten Linie, die vom Schnittpunkt des ersten Drahts 11 mit der Querschnittsebene QSE zur Schlaufenspitze 21 bzw. zum Schnittpunkt zwischen der Schlaufenspitze 21 und der Längsschnittebene LSE verläuft, und der Längsschnittebene LSE aufgespannt wird. Der Durchschnittsflecht-winkel des gekrümmten Drahtabschnitts des Drahts 11 ist generell kleiner als der Flechtwinkel des zweiten Drahts 12 im Bereich des Längsendes 15.

Bei dem Ausführungsbeispiel gemäß Fig. 5 ist ein gerader Verlauf des zweiten Drahts 12 dargestellt. Es ist jedoch auch möglich, dass auch der zweite Draht 12 einen gekrümmten Verlauf insbesondere ähnlich wie der erste Draht 11, aufweist. Die Krümmung des zweiten Drahts 12 kann sich in dieselbe Richtung wie die Krümmung des ersten Drahts 11 erstrecken. Beispielsweise können beide Drähte 11, 12 einen konvex gekrümmten Verlauf aufweisen. Alternativ können beide Drähte 11, 12 einen konkav gekrümmten Verlauf aufweisen. Zumindest einer der beiden Drähte 11, 12 ist stärker gekrümmt als der andere Draht 12, 11. Damit wird erzielt, dass sich die beiden Drähte 11, 12 in Richtung der Schlaufenspitze 18 einander annähern. Die beiden Drähte 11, 12 konvergieren zueinander. Es ist auch denkbar, dass die Krümmungsrichtung der beiden Drähte 11, 12 unterschiedlich ist. Beispielsweise kann der erste Draht 11 einen konkav gekrümmten Verlauf aufweisen und der zweite Draht 12 einen konvex gekrümmten Verlauf. Entsprechend lässt sich sowohl für den ersten Draht 11, als auch für den zweiten Draht 12 ein Durchschnittsflechtwinkel ermitteln, der zwischen der Längsschnittebene LSE und einer gedachten Linie aufgespannt ist, wobei sich die gedachte Linie von der Schlaufenspitze 21 zum Schnittpunkt des jeweiligen Drahts 11, 12 mit der Querschnittebene QSE geradlinig erstreckt. Die beiden Durchschnittsflechtwinkel des ersten Drahts 11 und des zweiten Drahts 12 sind unterschiedlich, so dass sich die beiden Drähte 11, 12 in Richtung zur Schlaufenspitze 21 einander annähern.

Die Differenz zwischen dem Durchschnittsflechtwinkel des ersten Drahts 11 und dem Flechtwinkel bzw. Durchschnittsflechtwinkel des zweiten Drahts 12 im Bereich des Längsendes 15 beträgt vorzugsweise wenigstens 2 Grad, insbesondere wenigstens 4 Grad, insbesondere wenigstens 6 Grad, insbesondere wenigstens 8 Grad, insbesondere wenigstens 10 Grad, insbesondere wenigstens 12 Grad, insbesondere wenigstens 15 Grad, insbesondere wenigstens 20 Grad. Insgesamt können die beiden Drähte 11, 12 jeweils einen Flechtwinkel bzw. Durchschnittsflechtwinkel zwischen 30 Grad und 75 Grad, insbesondere zwischen 40 Grad und 60 Grad, insbesondere zwischen 50 Grad und 55 Grad, insbesondere 55 Grad, aufweisen.

Die Drähte 11, 12 können innerhalb des Gittergeflechts 10 in unterschiedlichen Flechtarten geführt sein. Beispielsweise kann jeweils ein Draht 11, 12 einen weiteren Draht überkreuzen. Insbesondere kann eine 1-über-1-Flechtung vorgesehen sein. Ferner kann das Gittergeflecht eine 1-über-2-Flechtung oder 2-über-2-Flechtung umfassen. Bei einer 1-über-2-Flechtung überkreuzt jeweils ein Draht 11, 12 zwei weitere Drähte. Bei einer 2-über-2-Flechtung werden jeweils zwei weitere Drähte durch zwei Drähte 11, 12 überkreuzt. Eine kleine Anzahl von überkreuzten Drähten (1-über-1-Flechtung) verbessert die Stabilität des Gittergeflechts. Eine größere Anzahl von überkreuzten Drähten (1-über-2-Flechtung, 2-über-2-Flechtung) verbessert hingegen die Crimpbarkeit bzw. Komprimierbarkeit des Gittergeflechts 10 und verringert gleichzeitig die Empfindlichkeit des Gittergeflechts 10 gegenüber Verzerrungen bzw. Tor-sion. Es ist möglich, dass sich die Flechtart beim Übergang vom Schlaufenbereich am axialen Ende zum Geflechtinneren ändert. Im Bereich des Längsendes 15 kann das Gittergeflecht 10 also eine andere Flechtart aufweisen als im Geflechtinneren. Die Flechtart kann sich insbesondere am ersten Kreuzungspunkt 16 ändern, der den Schlaufen 13, 14 bzw. Dem axialen Ende des Geflechts am Nächsten ist. Insbesondere kann sich die Flechtart im Bereich der Schlaufenendpunkte 17 ändern. Beispielsweise kann das Gittergeflecht 10 im Bereich der Schlaufen 13, 14 eine 1-über-2-Flechtung, eine 2-über-2-Flechtung oder eine 4-über-4-Flechtung aufweisen. Im Geflechtinneren liegt hingegen eine 1-über-1-Flechtung vor, so dass das Geflechtinnere eine höhere Stabilität als das Längsende 15 aufweist. Das Längsende 15 des Gittergeflechts 10, also der Bereich der Schlaufen 13, 14 weist also eine größere Flexibilität als das Geflechtinnere auf. Dabei bezieht sich die Flexibilität auf die Möglichkeit der einzelnen Drähte 11, 12, sich gegeneinander zu bewegen. Mit anderen Worten weist das Längsende 15 des Gittergeflechts bei der vorgenannten Konfiguration eine höhere Bewegungsfreudigkeit auf als das Geflechtinnere.

Alternativ kann vorgesehen sein, dass das Gittergeflecht 10 in Richtung des Längsendes 15, also in Richtung der Schlaufen 13, 14, stabiler wird. Beispielsweise kann im Bereich der Schlaufen 13, 14 eine 1-über-1-Flechtung vorgesehen sein, wogegen im Geflechtinneren eine 1-über-2-Flechtung, eine 2-über-2-Flechtung oder eine 4-über-4-Flechtung bereitgestellt wird. Das hat den Vorteil, dass sich die Schlaufen 13, 14 der Expansion des Gittergeflechts 10 stärker widersetzen, also die einzelnen Drähte 11, 12 im Bereich der Schlaufen 13, 14 weniger bewegungsfreudig als im Geflechtinneren sind. Mit anderen Worten weisen die Mehrfachschlaufen am Längsende des Gittergeflechts 10 eine höhere Steifigkeit auf als die Maschen im Gittergeflecht 10. Im Allgemeinen hat es sich als vorteilhaft erwiesen, wenn die Flechtart, die am Längsende 15 des Gittergeflechts 10 eingestellt ist, wenigstens bis zu einer ersten Überkreuzung bzw. einem ersten Kreuzungspunkt 16, insbesondere bis zu einem zweiten, insbesondere bis zu einem dritten, insbesondere bis zu einem vierten oder über weitere Überkreuzungen bzw. Kreuzungspunkte 16 ausgehend vom Längsende 15 beibehalten wird. Im weiteren Verlauf in Richtung des Geflechtinneren kann sich die Flechtart ändern.

Bei den vorbeschriebenen Ausführungsbeispielen ist vorgesehen, dass die medizinische Vorrichtung ein Gittergeflecht 10 mit einem Längsende 15 aufweist, wobei das Längsende 15 Doppelschlaufen umfasst, die aus jeweils einer ersten Schlaufe 13 und einer zweiten Schlaufe 14 gebildet sind. Es ist allerdings auch möglich, mehr als zwei Schlaufen 13, 14 im Längsende des Gittergeflechts 10 im expandierten Zustand zu überlagern. Beispielsweise können drei, vier, fünf oder mehr Schlaufen 13, 14 vorgesehen sein, die im expandierten Zustand des Gittergeflechts 10 zumindest abschnittsweise deckungsgleich aufeinander angeordnet sind. Die einzelnen Schlaufen 13, 14 derartiger Mehrfachschlaufen können im komprimierten Zustand des Gittergeflechts 10 voneinander beabstandet angeordnet sein.

Es ist ferner möglich, dass die medizinische Vorrichtung ein Gittergeflecht 10 umfasst, das an einem Längsende 15 Mehrfachschlaufen aufweist. Insbesondere können an einem Längsende 15 ausschließlich Doppelschlaufen oder Mehrfachschlaufen vorliegen. Das bedeutet, dass alle Drähte 11, 12 an einem Längsende 15 in Schlaufen 13, 14 übergehen, wobei jeweils wenigstens zwei Schlaufen 13, 14 in Längsrichtung des Gittergeflechts 10 zumindest im komprimierten Zustand des Gittergeflechts 10 angeordnet sind.

Ein gegenüberliegendes Längsende des Gittergeflechts 10 kann eine andere Struktur bzw. Form umfassen. Beispielsweise kann das andere Längsende des Gittergeflechts 10 eine umlaufende, insbesondere schräge, Kante bilden, so dass die medizinische Vorrichtung nach Entlassung aus einem Zuführsystem einfach und mit wenig Kraftaufwand wiedereinziehbar ist. Ein derartiges wiedereinziehbares Geflechtende ist beispielsweise in der nachveröffentlichten DE 10 2009 056 450.0, insbesondere auf Seiten 15-24 in Verbindung mit Figuren 1-4, beschrieben, die auf dieselbe Anmelderin zurückgeht und deren Offenbarungsgehalt vollumfänglich in die vorliegende Anmeldung aufgenommen wird. Im Zusammenhang mit der vorgenannten DE 10 2009 056 450.0 wird also im Rahmen der Anmeldung auch eine medizinische Vorrichtung offenbart und beansprucht, die ein Längsende aufweist, das mehrere Mehrfachschlaufen umfasst, und ein weiteres Längsende, das gemäß DE 10 2009 056 450.0 ausgebildet ist. Beide Längsenden, also sowohl mit Mehrfachschlaufen gemäß Figuren 1, 2 und 5, als auch mit einer umlaufenden Kante gemäß DE 10 2009 056 450.0, sind atraumatisch.

Die Herstellung des Gittergeflechts 10 erfolgt auf einem Flechtdorn, der mehrere Pins umfasst, um die die einzelnen Drähte 11, 12 gewickelt werden. Das Längsende 15 des Gittergeflechts 10 wird gebildet, indem die ersten und zweiten Drähte 11, 12 um mehrere auf dem Umfang des Flechtdorns gleichmäßig beabstandet angeordnete Pins gelegt werden. Dabei werden jeweils zwei Drähte, ein erste Draht 11 und ein zweiter Draht 12, um einen Pin gelegt, so dass über das Längsende 15 des Gittergeflechts 10 mehrere Doppelschlaufen gebildet sind. Es ist auch möglich, mehr als zwei Drähte über einen einzigen Pin zu legen, so dass Mehrfachschlaufen gebildet werden, die mehr als zwei Drähte bzw. Einzelschlaufen umfassen.

Das Gittergeflecht 10 kann insgesamt aus mehreren Drähten gebildet sein, die durch Umlenken in den Schlaufen 13, 14 jeweils zwei Einzeldrahtabschnitte bilden. Zur Bildung von Doppelschlaufen weist der Flechtdorn, auf dem das Gittergeflecht 10 hergestellt wird, eine Anzahl von Pins auf, die einem Viertel der Drahtanzahl entspricht. Bei einem Gittergeflecht 10, das aus insgesamt 48 Einzeldrähten bzw. Einzeldrahtabschnitten gebildet ist, erfolgt die Herstellung auf einem Flechtdorn, der 12 Pins aufweist, so dass sich insgesamt 24 Schlaufen, also 12 Doppelschlaufen bilden. Jeweils zwei Einzeldrähte bzw. Einzeldrahtabschnitte sind in einer Schlaufenspitze 21 einstückig miteinander verbunden, wobei die Einzeldrähte in unterschiedlichen Windungsrichtungen um die Längsachse des Gittergeflechts 10 umlaufen. Ein einziger am Längsende des Gittergeflechts 10 umgelenkter Draht bildet also zwei Einzeldrähte bzw. Einzeldrahtabschnitte. Vorzugsweise weist das Gittergeflecht 10 wenigstens 12 Einzeldrähte auf, so dass sich 6 Schlaufen bzw. drei Doppelschlaufen bilden lassen. Besonders bevorzugt ist eine Anzahl von wenigstens 16, insbesondere wenigstens 24, insbesondere wenigstens 32, insbesondere wenigstens 40, insbesondere wenigstens 48 Einzeldrähten. Bei 48 Einzeldrähten lassen sich insgesamt 24 Schlaufen, also 12 Doppelschlaufen bilden. Die einzelnen Einzeldrähte werden innerhalb des Gittergeflechts miteinander verflochten, wobei eine der zuvor beschriebenen Flechtarten zum Einsatz kommt. Die Herstellung des Gittergeflechts 10 erfolgt in einem expandierten Zustand, wobei die Schlaufen 13, 14, die um die Pins des Flechtdorns gelegt sind, aufeinander aufliegend angeordnet werden, wie beispielsweise in Fig. 5 gezeigt.

Das Gittergeflecht 10 kann aus mehreren Drähten gebildet sein, die unterschiedliche Drahtstärken aufweisen. Insbesondere kann vorgesehen sein, dass eine Mehrfachschlaufe bzw. Doppelschlaufe wenigstens zwei Drähte 11, 12 umfasst, wobei einer der Drähte 11, 12 eine größere Drahtstärke als der anderen Draht 12, 11 aufweist. Auf diese Weise wird eine Funktionstrennung erreicht. Der Draht 11, 12 mit der größeren Drahtstärke, also mit dem größeren Drahtquerschnitt, bewirkt eine erhöhte Radialkraft, wogegen der Draht 12, 11 mit relativ kleinerer Drahtstärke, also mit geringerem Drahtquerschnitt, die Aufgabe übernimmt, die Feinmaschigkeit des Gittergeflechts 10 zu erhöhen. Es kann vorgesehen sein, dass das Gittergeflecht 10 eine, insbesondere ausschließlich eine, Doppelschlaufe bzw. Mehrfachschlaufe aufweist, die wenigstens zwei Drähte 11, 12 mit unterschiedlichen Drahtstärken bzw. Drahtquerschnitten aufweist. Insbesondere kann das Gittergeflecht 10 wenigstens eine, insbesondere wenigstens 2, insbesondere wenigstens 3, insbesondere mehr als 3 Mehrfachschlaufen bzw. Doppelschlaufen aufweisen, die aus zwei Drähten 11, 12 gebildet sind, die unterschiedliche Drahtstärken bzw. Drahtquerschnitte umfassen. Die Mehrfachschlaufen bzw. Doppelschlaufen mit Drähten 11, 12, die unterschiedliche Drahtstärken aufweisen, können regelmäßig über das Längsende des Gittergeflechts 10 verteilt sein. Beispielsweise kann jede zweite Doppelschlaufe bzw. Mehrfachschlaufe zwei Drähte 11, 12 aufweisen, die unterschiedliche Drahtstärken umfassen. Andere Muster sind möglich. Vorteilhaft ist auch eine Ausgestaltung, bei der alle Mehrfachschlaufen bzw. Doppelschlaufen eines Längsendes des Gittergeflechts 10 aus Drähten 11, 12 gebildet sind, die unterschiedliche Drahtstärken aufweisen.

Vorzugsweise ist vorgesehen, dass der zweite Draht 12, also derjenige Draht 12, der im komprimierten Zustand des Gittergeflechts 10 die zweite, äußere Schlaufe 14 bildet, einen größeren Drahtquerschnitts bzw. eine größere Drahtstärke als der erste Draht 11 aufweist. Mit anderen Worten ist die im komprimierten Zustand des Gittergeflechts 10 axial äußere Schlaufe 14 durch einen Draht 12 gebildet, der im Vergleich zu einem Draht 11, der eine axial innere Schlaufe 13 derselben Doppelschlaufe bzw. Mehrfachschlaufe bildet, einen größeren Drahtquerschnitt bzw. eine größere Drahtstärke aufweist. Insbesondere ist an einem Längsende 15 des Gittergeflechts 10 wenigstens eine Doppelschlaufe oder Mehrfachschlaufe angeordnet, die aus wenigstens zwei Drähten 11, 12 gebildet ist, wobei der Draht 12, der den vergleichsweise größeren Drahtquerschnitt aufweist, im komprimierten Zustand des Gittergeflechts 10 axial außen angeordnet ist, also weiter über das Gittergeflecht 10 vorsteht als ein oder mehrere axial innere Drähte 11 derselben Doppelschlaufe bzw. Mehrfachschlaufe.

An einem Längsende 15 des Gittergeflechts 10 können wenigstens zwei, insbesondere wenigstens drei, insbesondere wenigstens vier, insbesondere wenigstens sechs, Schlaufen bzw. Mehrfachschlaufen vorgesehen sein, die durch Drähte 11, 12 mit unterschiedlichen Drahtstärken gebildet sind, wobei der relativ dickere Draht im komprimierten Zustand des Gittergeflechts 10 axial außen angeordnet ist. Weitere Doppelschlaufen bzw. Mehrfachschlaufen können aus einzelnen Schlaufen 13, 14 gebildet sein, deren Drähte 11, 12 dieselbe Drahtstärke aufweisen. Dies gilt grundsätzlich für jede beliebige Anzahl von Doppelschlaufen bzw. Mehrfachschlaufen an einem Längsende des Gittergeflechts 10. Insbesondere können mehr als sechs Doppelschlaufen bzw. Mehrfachschlaufen an einem Längsende 15 des Gittergeflechts 10 vorgesehen sein, wobei ein Teil der Mehrfachschlaufen bzw. Doppelschlaufen, insbesondere eine oder mehrere Doppelschlaufen bzw. Mehrfachschlaufen eine äußere Schlaufe 14 umfassen, deren Draht 12 eine größere Drahtstärke bzw. einen größeren Drahtquerschnitt als ein Draht 11 einer axial inneren Schlaufe derselben Doppelschlaufe bzw. Mehrfachschlaufe aufweist.

In einer speziellen Ausgestaltung ist vorgesehen, dass an dem Längsende 15 des Gittergeflechts 10 insgesamt 12 Doppelschlaufen bzw. Mehrfachschlaufen gebildet sind, die insgesamt 24 erste und zweite Schlaufen 13, 14 umfassen, von denen wenigstens eine, insbesondere wenigstens zwei, insbesondere wenigstens drei, insbesondere wenigstens vier, insbesondere wenigstens sechs, Doppelschlaufen bzw. Mehrfachschlaufen erste und zweite Schlaufen 13, 14 aufweisen, wobei der zweite Draht 12, der die zweite Schlaufe 14 bildet, einen größeren Drahtquerschnitt als der erste Draht 11 aufweist, der die erste Schlaufe 13 bildet. Dabei ist die zweite Schlaufe 14 im komprimierten Zustand des Gittergeflechts 10 axial weiter außen angeordnet bzw. weiter vom Geflechtinneren entfernt angeordnet als die erste Schlaufe 13.

Konkret das Gittergeflecht 10 an einem Längsende 15 zwölf Doppelschlaufen bzw. Mehrfachschlaufen umfassen, die aus zwei Schlaufen 13, 14 gebildet sind, wobei eine der beiden Schlaufen 13, 14 einen Draht umfassen, der einen größeren Drahtquerschnitt als die Drähte der übrigen Schlaufen 14, 13 aufweist. Die Schlaufe 13, 14, die einen Draht 11, 12 mit einem relativ größeren Querschnittsdurchmesser aufweist, wird im folgenden als Verstärkungsschlaufe bezeichnet.

Vorteilhafterweise weisen zwei von insgesamt zwölf Doppelschlaufen weisen jeweils eine Verstärkungsschlaufe auf. Die übrigen zehn Doppelschlaufen sind hingegen aus anderen Schlaufen gebildet, deren Drähte jeweils denselben Querschnittsdurchmesser aufweisen. In den beiden Doppelschlaufen, die eine Verstärkungsschlaufe aufweisen, ist vorzugsweise die äußere Schlaufe, also die das Längsende 15 des Gittergeflechts 10 begrenzende Schlaufe, als Verstärkungsschlaufe ausgebildet. Dabei weist der Draht, der die Verstärkungsschlaufe bildet, vorzugsweise einen Querschnittsdurchmesser von 85 µm auf. Die Drähte der anderen Schlaufen umfassen vorzugsweise einen Querschnittsdurchmesser von 35 µm.

Mit anderen Worten umfasst das Längsende 15 des Gittergeflechts 10 vorzugsweise zwölf Doppelschlaufen, die jeweils eine innere Schlaufe, also eine der Gittergeflechtmitte nähere Schlaufe, aufweisen, deren Draht einen Querschnittsdurchmesser von 35 µm aufweist. Zwei der zwölf Doppelschlaufen weisen jeweils eine äußere Schlaufe auf, deren Draht einen Querschnittsdurchmesser von 85 µm umfasst. Der Draht der jeweils äußeren Schlaufe der übrigen zehn Doppelschlaufen weist hingegen denselben Querschnittsdurchmesser wie der Draht der inneren Schlaufen, also 35 µm, auf.

Insgesamt weist das Längsende 15 in der zuvor beschriebenen Variante 24 einzelne Schlaufen 13, 14 auf, wobei zwei der einzelnen Schlaufen 13, 14, die unterschiedlichen Mehrfachschlaufen zugeordnet sind, als Verstärkungsschlaufen ausgebildet sind.

Grundsätzlich ist es vorteilhaft, eine geringe Anzahl von Verstärkungsschlaufen vorzusehen, wobei jedoch der Querschnittsdurchmesser der Drähte, die die Verstärkungsschlaufen bilden, relativ groß gewählt ist. Auf diese Weise lässt sich die Radialkraft steigern.

Es ist bevorzugt, wenn mindestens eine, insbesondere mindestens zwei, insbesondere mindestens drei, insbesondere mindestens vier, insbesondere mindestens sechs, Mehrfachschlaufen von insgesamt zwölf Mehrfachschlaufen, die ein Längsende 15 eines Gittergeflechts 10 bilden, jeweils eine Verstärkungsschlaufe aufweisen, oder generell aus Schlaufen 13, 14 gebildet sind, deren Drähte 11, 12 unterschiedliche Querschnittsdurchmesser aufweisen. Dadurch wird ein besonders homogenes Gittergeflecht 10 bezogen auf die Verteilung der Radialkraft bereitgestellt. Generell ist bevorzugt, dass das Verhältnis zwischen der Anzahl der Mehrfachschlaufen mit einer oder mehreren Verstärkungsschlaufen zur Gesamtanzahl der Mehrfachschlaufen mindestens 1/12, insbesondere mindestens 1/6, insbesondere mindestens 1/4, insbesondere mindestens 1/3, insbesondere mindestens 1/2 beträgt.

Alternativ kann vorgesehen sein, dass höchstens sechs, insbesondere höchstens vier, insbesondere höchstens drei, insbesondere höchstens zwei, insbesondere höchstens eine Mehrfachschlaufe einer Gesamtzahl von zwölf Mehrfachschlaufen an einem Längsende 15 des Gittergeflechts 10 aus Drähten mit unterschiedlichem Querschnittsdurchmesser gebildet ist. Mit anderen Worten weisen höchstens sechs, insbesondere höchstens vier, insbesondere höchstens drei, insbesondere höchstens zwei, insbesondere höchstens eine, Mehrfachschlaufe von insgesamt zwölf Mehrfachschlaufen eine oder mehrere Verstärkungsschlaufen auf. Durch die Begrenzung der Anzahl von Verstärkungsschlaufen wird ermöglicht, den Querschnittsdurchmesser des Drahts, der die Verstärkungsschlaufe bildet, zu erhöhen, ohne den Gesamtdurchmesser des Gittergeflechts 10 bzw. des Längsendes 15, insbesondere im komprimierten Zustand, negativ zu beeinflussen. Generell ist bevorzugt, dass das Verhältnis zwischen der Anzahl der Mehrfachschlaufen mit einer oder mehreren Verstärkungsschlaufen zur Gesamtanzahl der Mehrfachschlaufen höchstens 1/2, insbesondere höchstens 1/3, insbesondere höchstens 1/4, insbesondere höchstens 1/6, insbesondere höchstens 1/12 beträgt.

Es ist auch möglich, dass die Mehrfachschlaufen mehr als eine Verstärkungsschlaufe aufweisen. Insbesondere ist vorgesehen, dass das Gittergeflecht 10 insbesondere sechs, insbesondere höchstens vier, insbesondere höchstens drei, insbesondere höchstens zwei, insbesondere höchstens eine, Mehrfachschlaufe aufweist, die jeweils wenigstens eine Verstärkungsschlaufe, insbesondere ausschließlich eine einzige Verstärkungsschlaufe, aufweisen.

Das Verhältnis der Anzahl von Verstärkungsschlaufen zu der Gesamtanzahl der Schlaufen pro Mehrfachschlaufe kann höchstens 1/2, insbesondere höchstens 1/4, insbesondere höchstens 1/6 betragen. Die Untergrenze beträgt eine einzige Verstärkungsschlaufe.

Das Verhältnis der Anzahl von Verstärkungsschlaufen zu der Gesamtanzahl der Schlaufen beträgt in einer bevorzugten Variante höchstens 1:2, insbesondere höchstens 1:4, insbesondere höchstens 1:6, insbesondere höchstens 1:8, insbesondere höchstens 1:10, insbesondere höchstens 1:12, insbesondere höchstens 1:16, insbesondere höchstens 1:18, insbesondere höchstens 1:24. Beispielsweise können an dem Längsende 15 des Gittergeflechts 10 ausschließlich Doppelschlaufen vorgesehen sein, die jeweils eine einzige Verstärkungsschlaufe umfassen, um ein Verhältnis zwischen der Anzahl der Verstärkungsschlaufen und der Gesamtanzahl an Schlaufen von 1:2 einzustellen. Im Allgemeinen ist es bevorzugt, wenn die Verstärkungsschlaufen jeweils die äußeren Schlaufen einer Mehrfachschlaufe bilden.

Die Verstärkungsschlaufen des Gittergeflechts 10 sind jeweils durch Drähte gebildet sein, die denselben Querschnittsdurchmesser aufweisen.

Insbesondere ist vorgesehen, dass die Drähte der anderen Schlaufen, die gemeinsam mit wenigstens einer Verstärkungsschlaufe eine Doppel- oder Mehrfachschlaufe bilden, denselben Querschnittsdurchmesser aufweisen wie die Drähte der anderen Schlaufen, die entweder als Einzelschlaufen am Längsende 15 vorliegen oder eine Mehrfachschlaufe bilden, die ausschließlich aus anderen Schlaufen, also ohne eine Verstärkungsschlaufe, gebildet ist.

Die Drähte, die die Verstärkungsschlaufen bilden, weisen zumindest im Bereich der Verstärkungsschlaufe vorzugsweise einen Querschnittsdurchmesser von wenigstens 50 µm, insbesondere wenigstens 60 µm, insbesondere wenigstens 70 µm, insbesondere wenigstens 80 µm, insbesondere wenigstens 85 µm, insbesondere wenigstens 100 µm, insbesondere wenigstens 150 µm, auf. Die Drähte, die die anderen Schlaufen bilden, weisen hingegen vorzugsweise einen Querschnittsdurchmesser auf, der höchstens 70 µm, insbesondere höchstens 50 µm, insbesondere höchstens 40 µm, insbesondere höchstens 35 µm, insbesondere höchstens 30 µm, insbesondere höchstens 25 µm, insbesondere höchstens 20 µm, umfasst. Dabei ist vorteilhaft ein Verhältnis zwischen dem Querschnittsdurchmesser der Drähte, die die anderen Schlaufen bilden, und dem Querschnittsdurchmesser der Drähte, die die Verstärkungsschlaufen bilden, von höchstens 0,8, insbesondere höchstens 0,7, insbesondere höchstens 0,6, insbesondere höchstens 0,5, insbesondere höchstens 0,4, insbesondere höchstens 0,3, insbesondere höchstens 0,2, insbesondere höchstens 0,1, vorgesehen.

### Bezugszeichenliste

- 10: Gittergeflecht
- 11: erster Draht
- 12: zweiter Draht
- 13: erste Schlaufe
- 14: zweite Schlaufe
- 15: Längsende
- 16: Kreuzungspunkt
- 17: Schlaufenendpunkte
- 18: Kontaktbereich
- 19: Masche
- 19a: erste Masche
- 19b: zweite Masche
- 20: Endmasche
- 21: Schlaufenspitze

- LSE: Längschnittebene
- QSE: Querschnittsebene
- LR: Längsrichtung
- UR: Umfangsrichtung

## Patentansprüche

1. Medizinische Vorrichtung mit einem expandierbaren Gittergeflecht (10) aus sich überkreuzenden Drähten (11, 12), die an wenigstens einem Längsende (15) des Gittergeflechts (10) Schlaufen (13, 14) bilden,
**dadurch gekennzeichnet, dass**
ein erster Draht (11) eine erste Schlaufe (13) und ein zweiter Draht (12) eine zweite Schlaufe (14) bilden, wobei die erste Schlaufe und die zweite Schlaufe in einem expandierten Zustand des Gittergeflechts (10) zumindest abschnittsweise deckungsgleich aufeinander und in einem komprimierten Zustand des Gittergeflechts (10) voneinander beabstandet angeordnet sind.

2. Medizinische Vorrichtung nach Anspruch 1,
**dadurch gekennzeichnet, dass**
die erste Schlaufe (13) und die zweite Schlaufe (14) im komprimierten Zustand des Gittergeflechts (10) hintereinander angeordnet sind.

3. Medizinische Vorrichtung nach Anspruch 1 oder 2,
**dadurch gekennzeichnet, dass**
die Schlaufen (13, 14) einstückig durch jeweils einen einzigen Draht (11, 12) der am Längsende (15) des Gittergeflechts (10) umgelenkt ist, oder durch jeweils zwei Drähte (11, 12) gebildet sind, die am Längsende (15) des Gittergeflechts (10) miteinander verbunden sind.

4. Medizinische Vorrichtung nach einem der Ansprüche 1-3,
**dadurch gekennzeichnet, dass**
das Gittergeflecht (10) rotationssymmetrisch, insbesondere zylinderförmig, ausgebildet ist, wobei die Drähte (11, 12) spiralförmig um eine gemeinsame Rotationsachse gewunden sind.

5. Medizinische Vorrichtung nach einem der Ansprüche 1-4,
**dadurch gekennzeichnet, dass**
der erste Draht (11) und der zweite Draht (12) innerhalb des Gittergeflechts (10) parallel zueinander verlaufen.

6. Medizinische Vorrichtung nach einem der Ansprüche 1-5,
**dadurch gekennzeichnet, dass**
die Drähte (11, 12) im Bereich der Schlaufen (13, 14) jeweils mit weiteren Drähten Kreuzungspunkte (16) bilden, die Schlaufenpunkte (17) festlegen, wobei der Abstand der Schlaufenpunkte (17) der ersten Schlaufe (13) kleiner als der Abstand der Schlaufenpunkte (17) der zweiten Schlaufe (14) ist.

7. Medizinische Vorrichtung nach einem der Ansprüche 1-6,
**dadurch gekennzeichnet, dass**
die erste Schlaufe (13) und die zweite Schlaufe (14) eine Mehrfachschlaufe bilden, die weitere Schlaufen umfassen kann, wobei wenigstens eine Schlaufe (13) als eine Verstärkungsschlaufe ausgebildet ist.

8. Medizinische Vorrichtung nach Anspruch 7,
**dadurch gekennzeichnet, dass**
das Verhältnis der Anzahl der Mehrfachschlaufen mit mindestens einer Verstärkungsschlaufe zur Gesamtzahl der Mehrfachschlaufen höchstens 1/2, insbesondere höchstens 1/3, insbesondere höchstens 1/4, insbesondere höchstens 1/6, insbesondere höchstens 1/12 beträgt.

9. Medizinische Vorrichtung nach Anspruch 7,
**dadurch gekennzeichnet, dass**
das Verhältnis der Anzahl der Verstärkungsschlaufen zur Gesamtzahl der Schlaufen höchstens 1/2, insbesondere höchstens 1/4, insbesondere höchstens 1/6 beträgt.

## Claims

1. Medical device with an expandable mesh (10) of intersecting wires (11, 12), which form loops (13, 14) on at least one longitudinal end (15) of the mesh (10),
**characterised in that**
a first wire (11) forms a first loop (13) and a second wire (12) forms a second loop (14), wherein in an expanded state of the mesh (10) the first loop and the second loop are at least in sections arranged congruently on one another and in a compressed state of the mesh (10) are arranged at a distance from one another.

2. Medical device according to claim 1
**characterised in that**
the first loop (13) and the second loop (14) are arranged one behind the other in the compressed state of the mesh (10).

3. Medical device according to claim 1 or 2
**characterised in that**
the loops (13, 14) are formed in one piece by a single wire (11, 12) which is deflected at the longitudinal end (15) of the mesh (10), or by two wires (11, 12) which are connected at the longitudinal end (15) of the mesh (10).

4. Medical device according to any one of claims 1-3
**characterised in that**
the mesh (10) is rotationally symmetrical, more particular cylindrical in design, wherein the wires (11, 12) are wound in a spiral manner around a common axis of rotation.

5. Medical device according to any one of claims 1-4
**characterised in that**
the first wire (11) and the second wire (12) run in parallel to each other within the mesh (10).

6. Medical device according to any one of claims 1-5
**characterised in that**
the wires (11, 12) in the region of the loops (13, 14) each form intersections (16) with other wires which define loop points (17), wherein the spacing of the loop points (17) of the first loop (13) is smaller than the spacing of the loop points (17) of the second loop (14).

7. Medical device according to any one of claims 1-6
**characterised in that**
the first loop (13) and the second loop (14) form a multiple loop which can comprise further loops, wherein at least one loop (13) is designed as a reinforcing loop.

8. Medical device according to claim 7
**characterised in that** the ratio of the number of multiple loops with at least one reinforcing loop to the total number of multiple loops is at most 1/2, more particularly at most 1/3, more particularly at most 1/4, more particularly at most 1/6, more particularly at most 1/12.

9. Medical product according to claim 7
**characterised in that** the ratio of the number of reinforcing loops to the total number of loops is at most 1/2, more particularly at most 1/4, more particularly at most 1/6.

## Revendications

1. Dispositif médical comprenant un treillis expansible (10) de fils se croisant (11, 12) qui forment des passants (13, 14) sur au moins une extrémité longitudinale (15) du treillis (10),
**caractérisé en ce qu'**un premier fil (11) forme un premier passant (13) et un second fil (12) forme un second passant (14), sachant que le premier passant et le second passant sont disposés en se recouvrant au moins par tronçons lorsque le treillis (10) est dans l'état expansé et sont disposés à distance l'un de l'autre lorsque le treillis (10) est dans l'état comprimé.

2. Dispositif médical selon la revendication 1, **caractérisé en ce que** le premier passant (13) et le second passant (14) sont disposés l'un derrière l'autre lorsque le treillis (10) est dans l'état comprimé.

3. Dispositif médical selon la revendication 1 ou 2, **caractérisé en ce que** les passants (13, 14) sont formés de façon monobloc par respectivement un seul fil (11, 12) qui est dévié sur l'extrémité longitudinale (15) du treillis (10), ou par respectivement deux fils (11, 12) qui sont reliés entre eux sur l'extrémité longitudinale (15) du treillis (10).

4. Dispositif médical selon l'une des revendications 1-3, **caractérisé en ce que** le treillis (10) est conçu symétrique en rotation, en particulier cylindrique, sachant que les fils (11, 12) sont enroulés en spirale sur un axe de rotation commun.

5. Dispositif médical selon l'une des revendications 1-4, **caractérisé en ce que** le premier fil (11) et le second fil (12) sont parallèles entre eux à l'intérieur du treillis (10).

6. Dispositif médical selon l'une des revendications 1-5, **caractérisé en ce que** les fils (11, 12) forment des points de croisement (16), avec respectivement d'autres fils au niveau des passants (13, 14), qui déterminent des points de passant (17), sachant que l'écart des points de passant (17) du premier passant (13) est inférieur à l'écart des points de passant (17) du second passant (14).

7. Dispositif médical selon l'une des revendications 1-6, **caractérisé en ce que** le premier passant (13) et le second passant (14) forment un passant multiple, qui peut inclure d'autres passants, sachant qu'au moins un passant (13) est conçu en tant que passant de renfort.

8. Dispositif selon la revendication 7, **caractérisé en ce que** le rapport du nombre de passants multiples avec au moins un passant de renfort au nombre total de passants multiples, fait au plus 1/2, en particulier au plus 1/3, en particulier au plus 1/4, en particulier au plus 1/6, en particulier au plus 1/12.

9. Dispositif selon la revendication 7, **caractérisé en ce que** le rapport du nombre de passants de renfort au nombre total de passants fait au plus 1/2, en particulier au plus 1/4, en particulier au plus 1/6.
